# EUROPEAN PATENT APPLICATION

(11) **EP 3 895 603 A1**
(43) Date of publication of application: **20.10.2021**
(21) Application number: 20169460.1
(22) Date of filing: 14.04.2020
(51) Int. Cl.: A61B 5/00, A61B 5/01, A61B 5/02, A61B 5/0404, A61B 5/145, A61B 5/1455, A61B 5/22

(54) **MONITORING DEVICE**

(71) Applicant: Biosensors Beyond Borders Ltd, London WC1B 3RE (GB)
(72) Inventor: Bozoky, Zoltan Egon, London W2 4RL (GB); Lafferty-Smith, Erin, Beckenham BR2 4HJ (GB); Schmit, Claude, 5425 Gostingen (LU); Gaifulina, Riana, Mill Hill NW7 1SE (GB); Taylor, Matthew, Tunbrudge Wells TW4 QUE (GB)
(74) Representative: Dehns

(57) **Abstract**

A monitoring device 100 for non-invasive and/or minimally invasive measurement of clinically useful patient data is provided. The monitoring device 100 comprises: (a) one or more sensors 101 for measuring mechanical, electrical, biochemical and/or molecular data; and (b) one or more sensors 101 for measuring skin property data.

## Description

The present invention relates to the field of medical monitoring devices.

In settings where disease levels may be well above what is seen in other populations and that may lack doctors or nurses, patients often experience delayed diagnosis. There is a paucity of screening tools that, when coupled with delayed presentation, means that patients who are ill, or are becoming ill, are often missed or are not treated appropriately.

According to a first aspect of the invention, there is provided a monitoring device for measuring patient data, the monitoring device comprising: (a) one or more sensors for measuring mechanical, electrical, biochemical, and/or molecular data; and (b) one or more sensors for measuring skin property data.

Patient data may comprise clinically useful data (and, in this case, particularly data that may be measured, e.g. using one or more sensors). The patient data may be data which could be useful (e.g. possibly in combination with other data) for clinical purposes such as assessing or detecting patient health.

As such, a single device can be provided which comprises sensors for measuring (various) patient or clinically useful data.

The patient data may include at least one of mechanical, electrical, biochemical and/or molecular data, and preferably at least two, three or all four of these data types. Preferably, the monitoring device comprises one or more sensors for measuring two, three or four of mechanical, electrical, biochemical and/or molecular data. This can provide a greater range of clinically useful (patient) data for subsequent analysis and interpretation.

In a hospital or modern clinic setting, it is usually not that difficult or inconvenient to provide separate devices with different sensors for making different clinically useful measurements (e.g. for measuring different kinds of patient data). However, operating these devices may require specific training for each device. In addition, further training is typically required in order to analyse and interpret the measured data and to provide an indication of a meaning or significance of the measured data. In many situations, such as in remote locations, low- or middle-income countries, low resource settings, or places where trained operators are not available, providing such separate devices with different sensors can be impractical, challenging to use (e.g. without adequate or extensive training) and costly.

However, the present invention may provide a single device with multiple mechanical, electrical, biochemical and/or molecular sensors, in combination with one or more sensors for measuring skin property data, which single device can be much easier to transport and operate than existing separate devices while increasing access to the technology for patient benefit.

Providing a monitoring device with one or more sensors for measuring skin property data can allow for skin property data to be measured which, in turn can allow for other (e.g. optical) measurements (e.g. made by other sensors) to be calibrated or adjusted for based on skin property data. This can allow for more accurate analysis and interpretation of patient data.

The one or more sensors for measuring mechanical, electrical, biochemical and/or molecular data, and/or the one or more sensors for measuring skin property data are preferably arranged to measure such data from a patient non-invasively (or minimally invasively). As such, clinically useful data may be measured from a patient non-invasively (or minimally invasively), i.e. without the need for invasive tests.

One or more of the one or more sensors for measuring mechanical, electrical, biochemical and/or molecular data, and/or one or more of the one or more sensors for measuring skin property data is/are preferably arranged to measure such data directly and/or data from which (further) such data may be determined.

One or more of the one or more sensors for measuring mechanical data may be arranged to measure (or measure data from which may be determined) haemodynamic data such as blood pressure, blood velocity, blood flow rate and/or relative blood flow.

One or more of the one or more sensors for measuring mechanical, electrical and/or molecular data may be arranged to measure cardiovascular data such as blood pressure, oxygen saturation, haemoglobin concentration, haemoglobin derivative(s), haematocrit, perfusion and/or circulation. Measuring blood pressure may comprise measuring (or determining from measured data) the timing and/or magnitude of the arterial pressure waveform.

One or more of the one or more sensors for measuring mechanical and/or molecular data may be arranged to measure haemorheological data such as blood clotting, platelet adhesion, platelet rigidity, and/or blood viscosity.

Such data as described above may be useful for providing an indication of a patient's health.

One or more of the one or more sensors for measuring mechanical, electrical, biochemical and/or molecular data, and/or one or more of the one or more sensors for measuring skin property data is/are preferably arranged to measure patient data at a patient's lower arm (e.g. below the elbow), hand(s) and/or finger(s). Preferably, all of the sensors for measuring mechanical, electrical, biochemical and/or molecular data, and the one or more of the one or more sensors for measuring skin property data is/are arranged to measure patient data at a patient's lower arm (e.g. below the elbow), hand(s) and/or finger(s). Such parts of a patient are relatively accessible and may be accessed in a discrete manner.

One or more sensors for measuring mechanical data preferably comprise mechanical and/or optical sensors, and preferably both.

One or more of the one or more sensors for measuring mechanical, electrical, biochemical and/or molecular data may comprise a blood pressure monitor, one or more optical sensors and/or one or more body composition sensors. Preferably two or, more preferably, all three of these kinds of sensors are provided in the monitoring device.

One or more of the one or more sensors for measuring skin property data preferably comprise(s) one or more optical sensors.

An optical sensor (e.g. as used in the monitoring device) may be an optical sensor for performing (e.g. UV, visible, near-infrared, mid-infrared, and/or far-infrared) transmission, absorption and/or reflectance spectroscopy, e.g. a spectrometer (spectroscopy sensor). An optical sensor may comprise a vibrational spectrometer. Such optical sensor(s) may comprise at least one light source (e.g. one or more LEDs) and at least one detector (e.g. suitable for detecting light emitted by the at least one light source). The at least one light source may comprise at least one LED, for example. The at least one light source may be arranged to emit white light and/or light at one or a plurality of wavelengths (e.g. including 785 nm). In one embodiment, a green light source (e.g. a green LED) is provided. In some embodiments, an optical sensor may comprise a light source (e.g. an LED) emitting white light, such as a single (e.g. LED) bulb or a single source emitting (only) white light. However, the optical sensor may alternatively comprise a plurality of light sources emitting light of different wavelengths (e.g. different colours).

An optical sensor may be arranged to operate at one or more wavelengths. This may provide a greater range of measurements/data, e.g. compared to an optical sensor operating at a single wavelength.

One or more of the one or more optical sensors is/are preferably arranged to measure mechanical and/or molecular data such as oxygen saturation, haemoglobin concentration, haemoglobin derivatives, haematocrit, platelet adhesion, platelet rigidity, blood clotting, and/or blood viscosity.

One or more of the one or more optical sensors is/are preferably arranged to measure biochemical data such as bilirubin level.

In some embodiments, no optical sensors are provided.

One or more sensors for measuring mechanical data is/are preferably suitable (e.g. adapted) for measuring mechanical data at a patient's lower arm (below the elbow), hand(s) and/or finger(s). For example, the one or more sensors for measuring mechanical data may be provided in the form of a sensor such as a finger sensor. In some embodiments, the one or more sensors for measuring mechanical data may be suitable (e.g. adapted) for measuring mechanical data at more than one location on a patient's lower arm (below the elbow), hand(s) and/or finger(s). The one or more sensors for measuring mechanical data is/are preferably arranged in the device such that, in use, it/they can sense, obtain, and/or measure data from a patient's lower arm (below the elbow), hand(s) and/or finger(s), and preferably (e.g. at least partially) from one or more locations on the lower arm (below the elbow), hand(s) and/or finger(s).

In particular, a blood pressure monitor is preferably arranged to measure mechanical data at a patient's lower arm (below the elbow), hand(s) and/or finger(s).

One or more sensors for measuring mechanical and/or electrical data is/are preferably arranged to measure mechanical and/or electrical data directly and/or data from which (further) mechanical and/or electrical data may be determined. For example, the one or more sensors for measuring mechanical and/or electrical data may be arranged to measure (or measure data from which may be determined) blood pressure, heart or pulse rate, perfusion and/or circulation.

Blood pressure e.g. as measured by (or determined from data measured by) the one or more sensors for measuring mechanical and/or electrical data preferably comprises information from or about the arterial pressure waveform.

Information from the arterial pressure waveform may be used to derive clinically useful data related to the timing and magnitude of the wave. As such, the device preferably comprises one or more processors arranged to determine (clinically useful) data related to the timing and/or magnitude of the arterial pressure wave from measurements made by the device.

One or more sensors for measuring mechanical and/or electrical data preferably comprise mechanical, electrical and/or optical sensors and/or one or more body composition sensors (or body composition monitors), and preferably two or three or these kinds of sensors. More than one optical sensor may be provided. The same optical sensor(s) may be used to measure mechanical and/or electrical as well as mechanical data. The same mechanical and/or electrical sensor(s) may be used to measure mechanical and/or electrical as well as mechanical data.

One or more sensors for measuring mechanical and/or electrical data are preferably arranged to sense, obtain and/or measure mechanical and/or electrical data at from a patient's lower arm (below the elbow), hands and/or finger(s).

One or more sensors for measuring mechanical and/or electrical data may be arranged to measure the arterial pressure waveform. However, preferably, one or more sensors for measuring mechanical and/or electrical data is/are arranged to measure clinically useful (patient) data related to the timing and/or magnitude of the arterial pressure wave. As such, the arterial pressure waveform may be measured at a patient's lower arm (below the elbow), hands and/or finger(s), eliminating a need for a patient's upper arm to be exposed (as is typically the case for such measurements), which some patients may find undesirable.

The arterial pressure waveform may comprise any measurements that provide clinically useful data related to the timing and/or magnitude of the arterial pressure wave, and preferably both of them.

One or more sensors for measuring mechanical and/or electrical data is/are preferably arranged to measure mechanical and/or electrical data directly and/or data from which (further) mechanical and/or electrical data may be determined.

One or more sensors for measuring the arterial pressure waveform is/are preferably provided in a cuff such as an inflatable cuff. Preferably, the cuff is arranged or configured in the device such that, in use, it is located at a patient's lower arm (below the elbow), hands and/or finger(s).

One or more sensors for measuring mechanical data may comprise a non-invasive blood pressure monitor. A non-invasive blood pressure monitor may be provided for measuring the arterial pressure waveform and clinically useful (patient) data related to the timing and magnitude of the wave.

One or more sensors for measuring mechanical data is/are preferably arranged to measure mechanical data directly and/or data from which (further) mechanical data may be determined. For example, the one or more sensors for measuring mechanical data may be arranged to measure (or measure data from which may determine) blood clotting and/or blood viscosity.

One or more sensors for measuring mechanical data preferably comprise one or more optical sensors. More than one optical sensor may be provided. The same optical sensor(s) maybe used to measure additional mechanical and/or electrical data.

The optical sensor(s) is/are preferably arranged to measure mechanical and/or electrical (or other clinically useful) data at least at a patient's lower arm (below the elbow), hands and/or finger(s).

The one or more sensors for measuring mechanical data preferably comprise one or more optical sensors such as described above.

At least one sensor for measuring mechanical data (e.g. comprising one or more optical sensors) may be provided in a scanner such as a finger scanner. The one or more sensors for measuring mechanical data is/are preferably arranged in the device such that, in use, it/they can sense, obtain and/or measure data from a patient's lower arm (below the elbow), hands and/or finger(s).

One or more optical sensors may be arranged to measure biochemical and/or molecular data (or data from which (further) biochemical and/or molecular data may be determined) such as haemoglobin concentration, haemoglobin derivatives (e.g. oxyhaemoglobin, deoxyhaemoglobin, carboxyhaemoglobin and methaemoglobin), haematocrit, platelet adhesion, and/or platelet rigidity; and/or biochemical and/or molecular data such as (e.g. transcutaneous) bilirubin level.

A separate optical sensor may be provided to measure biochemical and/or molecular data (or data from which biochemical and/or molecular data may be determined). For example, a separate (preferably non-invasive) optical sensor such as a non-invasive billirubinometer may be provided to measure patient data such as data related to (or for detecting) hepatic dysfunction.

One or more of the one or more body composition monitors is/are preferably arranged to measure bioimpedance, for example at a patient's hand(s), and preferably at both/each of a patient's hands, e.g. such that hand-to-hand bioimpedance may be measured.

One or more of the one or more body composition monitors is/are preferably arranged to measure (e.g. upper) body composition, (upper body) fat mass, lean mass, total body water, intracellular fluid, extracellular fluid, phase angle, breathing and/or clinically useful data related to the timing and/or magnitude of a pulse wave (e.g. an arterial pressure wave).

One or more of the one or more sensors for measuring skin property data is/are preferably arranged to measure skin phototype data, or data (e.g. such as reflectance spectroscopy data) from which skin phototype data may be determined. Skin phototype data and/or data from which skin phototype data may be determined may comprise data related to skin colour, skin pigmentation and/or presence of melanin. As such, the device may comprise one or more processors for determining skin phototype data from skin phototype data measured by the device. Thus, the clinically useful (patient) data preferably includes skin phototype data.

Skin phototype data may comprise any data which can provide an indication of a patient's skin phototype. For example, skin phototype data may comprise (or enable to be determined) a Fitzpatrick skin phototype class, such as Fitzpatrick skin phototype class I-II, Fitzpatrick skin phototype class III-IV, and/or Fitzpatrick skin phototype class V-VI.

One or more of the one or more sensors for measuring skin property data may additionally or alternatively be arranged to measure collagen, melanin and/or hydration (i.e. hydration of the skin), or data from which any or all of these properties may be determined.

One or more of the one or more sensors for measuring skin property data preferably comprise one or more optical sensors, such as described above. For example, one or more of the one or more sensors for measuring skin property data may include a UV, visible, near-infrared, mid-infrared, and/or far-infrared transmission, absorption and/or reflectance spectroscopy sensor(s), and/or may comprise one or more LED optical sensors such as a white, green and/or red LED optical sensor(s). Optical sensors of other wavelengths may additionally or alternatively be used.

Preferably, the one or more sensors for measuring skin property data are arranged in the device such that, in use, the one or more sensors for measuring skin property data emit and/or detect light onto/from one or more locations on a patient's lower arm (below the elbow), hands and/or finger(s) in order to measure the skin property data or data from which skin property data can be determined. Measuring skin property data or data from which skin property can be determined from a patient's lower arm (below the elbow), hands and/or finger(s) can help to provide an accurate indication of their skin property/properties data.

Preferably, the device comprises a plurality of sensors for measuring skin property data.

At least one sensor for measuring skin property data may be provided at one or more (or preferably each) location(s) (i.e. on a patient) where (e.g. other) light-based measurements are made. This can allow for some/all/any light-based measurement made by the device to be calibrated or adjusted according to a patient's skin property/properties at that location. A light-based measurement may be any measurement made by the device that involves directing a light source onto a patient. A location here refers to a location on a patient's body.

By including one or more sensors for measuring skin property data, or data from which skin property/properties data may be determined, in the monitoring device along with sensors for measuring other kinds of clinically useful (patient) data (e.g. as described above), skin property data may then be used in an analysis of and involving (the) other kinds of clinically useful data, allowing such an analysis to be improved compared, for example, with situations where skin property data is/are not available.

At least one sensor for measuring skin property data is preferably arranged in the device such that, in use, it can sense, obtain and/or measure data from a patient's lower arm (below the elbow), hands and/or finger(s).

One or more sensors for measuring skin property data preferably comprise one or more (and preferably two) optical sensors.

One or more sensors for measuring skin property data is/are preferably arranged to sense, obtain and/or measure skin property data at a patient's lower arm (below the elbow), hands and/or finger(s). Preferably, the one or more sensors for measuring skin property data are arranged to measure skin property data at more than one location on a patient's lower arm (below the elbow), hands and/or finger(s).

The device may further comprise one or more sensors for measuring (or measuring data from which may be determined) cardioelectrical and/or cardiomechanical data (and preferably both of these types of data).

The one or more sensors for measuring (cardiac) cardioelectrical and/or cardiomechanical data preferably comprise one or more electrical sensors. For example, cardioelectrical and/or cardiomechanical sensors may comprise a 1-lead, 6-lead and/or 12-lead ECG sensor or monitor.

The sensor for measuring cardioelectrical and/or cardiomechanical data may be arranged to measure a cardioelectrical and/or cardiomechanical waveform. This may be used to derive clinically useful (patient) data related to electrical impulses from the heart. As such, the device preferably comprises one or more processors arranged to determine clinically useful (patient) data, e.g. related to electrical impulses from the heart, from the cardioelectrical and/or cardiomechanical data measured by the device.

The one or more sensors for measuring cardioelectrical and/or cardiomechanical data is/are preferably arranged to measure cardioelectrical and/or cardiomechanical data at a patient's lower arm (below the elbow), hands and/or finger(s).

The device may further comprise a sensor for measuring mechanical data, such as a sensor for measuring muscle function and/or muscle strength data (e.g. continuous muscle function and/or (maximum) grip strength). This may be a mechanical and/or electrical sensor, for example. Such a sensor may be arranged to quantify the static force that can be measured, for example. Such a sensor is preferably arranged to measure mechanical data at a patient's lower arm (below the elbow), hands and/or finger(s). As such, the device preferably comprises one or more processors arranged to determine muscle function from mechanical data measured by the device.

The device may comprise a sensor for measuring biochemical and/or molecular data (or measuring data from which biochemical and/or molecular data may be determined) such as disease-specific variation in molecular states. This could be used to assess or detect life threatening infectious and/or cardiometabolic conditions. The sensor(s) for measuring biochemical and/or molecular data preferably comprises an optical sensor (e.g. as described above) such as a vibrational spectrometer. Preferably, this is a non-invasive and/or transcutaneous optical sensor.

A sensor for measuring biochemical and/or molecular data is preferably arranged to measure molecular data at a patient's lower arm (below the elbow), hands and/or finger(s).

The device may comprise a positioning system for determining a position of the monitoring device. Such position data, as obtained by a positioning system, may be used in analysis of clinically useful data measured by the device.

In preferred embodiments, the monitoring device further comprises at least one temperature sensor (e.g. a thermometer such as a digital thermometer). The at least one temperature sensor is/are preferably arranged in the device such that, in use, the temperature sensor(s) is/are arranged to sense, obtain and/or measure a patient's temperature at the skin and/or circulatory failure, particularly at their lower arm (below the elbow), hands and/or finger(s).

Preferably, at least two temperature sensors are provided. For example, at least two temperature sensors may be provided for measuring a patient's temperature at least two different locations, (e.g. at a finger and at an arm/forearm of a patient). This can allow a temperature gradient to be determined (e.g. from at least two temperature measurements made by the at least two temperature sensors).

One or more temperature sensors may be provided, e.g. in the form of a contact thermometer(s).

In preferred embodiments, the monitoring device further comprises a humidity (or moisture) sensor. The humidity sensor is preferably arranged in the device such that, in use, the humidity sensor is arranged to sense, obtain and/or measure a patient's skin humidity, particularly the skin humidity of their lower arm (below the elbow), hands and/or finger(s).

The humidity sensor may be provided in the form of a capacitance or corneometry sensor.

In preferred embodiments, the monitoring device further comprises at least one sensor for measuring fluorescence such as a UVA LED fluorescence sensor.

By including a sensor for measuring fluorescence data in the monitoring device, along with sensors for measuring other kinds of clinically useful (patient) data (e.g. as described above), fluorescence data may then be used in an analysis of and involving the other kinds of clinically useful data, allowing such an analysis to be improved compared, for example, with situations where fluorescence data are not available.

The at least one sensor for measuring fluorescence is preferably arranged in the device such that, in use, it can sense, obtain and/or measure data from a patient's lower arm (below the elbow), hands and/or finger(s).

The monitoring device preferably comprises one or more memories (and preferably a plurality of memories) for storing data measured by the monitoring device. Providing a plurality of memories can provide redundancy in the event of a memory failure.

The monitoring device preferably comprises one or more processors (and preferably a plurality of processors) arranged to (or comprising software configured to) analyse clinically useful (patient) data measured by the monitoring device and, preferably, to also provide an analysis result.

The monitoring device preferably comprises any or all of the following sensors at a high and/or at a low resolution.

A monitoring device (e.g. with sensors at a high and/or at a low resolution) may be provided on an online and/or offline basis.

A monitoring device may comprise any or all of the following sensors
- one or more optical, preferably multi-wavelength, sensors, such as a UV, visible, near-infrared, mid-infrared and/or far-infrared transmission, vibrational, absorption and/or reflectance spectroscopy sensor, a white (LED) optical sensor, and/or a green (LED) optical sensor
- a photoplethysmography (PPG) sensor such as a (green) LED PPG sensor
- an arterial pressure waveform monitor such as a non-invasive blood pressure monitor
- a cardioelectrical and/or cardiomechanical monitor such as a 1-lead, 6-lead or 12-lead ECG monitor
- one or more preferably digital temperature sensors (or thermometers) such as contact thermometers
- a humidity sensor
- a body composition sensor (or a pair of such sensors), such as a (pair of) (e.g. hand-to-hand) bioimpedance sensor(s)
- a preferably digital mechanical and/or electrical sensor for measuring muscle function, such as a strain gauge load cell.

The monitoring device preferably comprises sensors that, in use, are located at a patient's lower arm (below the elbow), hands and/or finger(s). Thus, the monitoring device may comprise a lower arm (below the elbow), hands and/or finger(s) monitoring part, which may comprise one or more sensors arranged or adapted for measuring or obtaining clinically useful (patient) data from a patient's lower arm (below the elbow), hands and/or finger(s). The lower arm (below the elbow), hands and/or finger(s) monitoring part preferably comprises one or more sensors for measuring mechanical, electrical, biochemical and/or molecular data, and/or skin property data (e.g. such as described above).

The lower arm (below the elbow), hands and/or finger(s) monitoring part may be provided in the form of a pedestal, such as a notched pedestal.

Preferably, the lower arm (below the elbow), hands and/or finger(s) monitoring part comprises a cover or shade (or a plurality or such covers or shades) arranged to shade one or more sensors of the lower arm (below the elbow), hands and/or finger(s) monitoring part from ambient light. This can assist in performing more accurate measurements.

The lower arm (below the elbow), hands and/or finger(s) monitoring part may be moveable, for example with respect to the rest of the monitoring device, e.g. such that it can accommodate different lower arm (below the elbow), hands and/or finger(s) sizes/lengths.

The lower arm (below the elbow), hands and/or finger(s) monitoring part preferably comprises one or more control units such as one or more microcontrol unit (MCU)s.

A plurality of control units may be provided, e.g. one for each sensor. A main or central control unit may be provided, e.g. for analysing or controlling data from each of the sensors.

The control unit of the lower arm (below the elbow), hands and/or finger(s)monitoring part preferably comprises memory for storing data obtained from or measured by the one or more sensors of the lower arm (below the elbow), hands and/or finger(s) monitoring part.

A plurality of memories may be provided, e.g. one for each sensor.

A memory may be associated with each control unit.

The control unit of the lower arm (below the elbow), hands and/or finger(s) monitoring part preferably comprises one or more processors (e.g. microprocessors) for analysing data obtained from or measured by the one or more sensors of the lower arm (below the elbow), hands and/or finger(s) monitoring part. For example, the one or more processors may be arranged (or comprise software configured) to analyse (or calibrate/adjust) mechanical, electrical, biochemical, molecular and/or skin property data from one or more sensors of the lower arm (below the elbow), hands and/or finger(s) monitoring part.

The monitoring device preferably may comprise a cuff, particularly an oscillometry or inflatable cuff. The cuff is preferably suitable for attachment around a patient's lower arm (below the elbow), hands and/or finger(s). The cuff preferably comprises at least one sensor for obtaining or measuring clinically useful data from a patient, such as at least one sensor for measuring mechanical and/or electrical data.

Preferably, the cuff is arranged in the device such that, in use, it is located around a patient's lower arm (below the elbow), hands and/or finger(s).

The cuff preferably comprises fastening means such as Velcro, a popper, a buckle or a button for fastening the cuff around an arm (e.g. a forearm) of a patient.

The monitoring device preferably comprises inflation means (e.g. a pump or bulb) for inflating the cuff, such that mechanical and/or electrical data can be measured or obtained from a patient. The inflation means are preferably manual inflation means. For example, a bulb may be provided for a user to squeeze in order to inflate the cuff manually. This can help to reduce the weight and power needs of the device, e.g. compared to providing an automatic/electric pump for inflating the cuff. However, alternatively, a powered or automatic inflation means, such as a pump with a motor could be provided.

The cuff preferably further comprises a control unit such as an MCU.

The control unit of the cuff preferably comprises memory for storing data obtained from or measured by the one or more sensors (e.g. at least one sensor for measuring mechanical and/or electrical data) of the cuff.

The control unit of the cuff preferably comprises one or more processors (e.g. microprocessors) for analysing data obtained from or measured by the one or more sensors of the cuff. For example, the one or more processors may be arranged (or comprise software configured) to analyse data from the one or more sensors for measuring mechanical and/or electrical data (such as the sensors described above).

The monitoring device preferably comprises at least one (e.g. main) processor, such as an MCU. The at least one (e.g. main) processor of the monitoring device is preferably arranged (or comprises software configured) to control or facilitate a measurement process performed with the device and/or to analyse clinically useful data obtained from such a measurement process (e.g. from one or more of the sensors of the monitoring device). The processor may be arranged to perform actions such as initiate and/or stop a measurement with one or more of the one or more sensors, store data from the one or more sensors, analyse data from the one or more sensors, cause data (or an indication of such data) from the one or more sensors to be displayed on a display, cause an indication of a measurement process (e.g. a status of a measurement process such as whether or not it is being performed, its time and/or its duration) to be displayed on a display, and/or cause a result of an analysis (or an indication or such a result) of data from the one or more sensors to be displayed on a display.

The monitoring device preferably further comprises communication means for facilitating communication, such as wired (e.g. USB) or wireless (e.g. via WiFi or a mobile data network), with a further device or system. As such, clinically useful (patient) data obtained from one or more measurement processes performed with the monitoring device may be transferred to a further device or system, e.g. for storage and/or further analysis. In addition, or alternatively, analysis software (e.g. updates to analysis software) and/or parameters (e.g. for use in analysis software) may be received at the monitoring device from a further device or system, for use in analysis of (e.g. future) data obtained with the monitoring device.

The monitoring device preferably comprises memory for storing data from a measurement process performed with the device. This may be in addition to the memory (memories) described above, thereby providing redundancy in case of a component failing.

In some embodiments, the monitoring device may further comprise a positioning system, such as a Global Positioning System (GPS), for determining a position of the monitoring device. Such a position may be used, for example, in analysis of data obtained with the device (e.g. analysis may be dependent on location). However, in alternative embodiments, no such positioning system is provided.

The monitoring device preferably further comprises a control means, such as a control panel, for controlling or operating the device.

The monitoring device preferably comprises a display, e.g. for displaying a status and/or result of a measurement process on the device and/or for providing a measurement instruction.

The control means and display may be provided in a single component, such as a touchscreen display panel. Alternatively, they may be provided separately.

The monitoring device may comprise a graphical user interface (GUI) for controlling and operating the device, e.g. initiating a measurement process and/or displaying a result of such a process. This may be a black and white or a colour GUI.

The control means could comprise a touchscreen, and/or one or more dials and/or buttons.

The monitoring device may comprise connection means for connection to a mains power supply, such as an AC mains supply. Preferably, the device may be used in multiple countries and may be powered by different mains supplies, e.g. 100-240 V, 13 A, 50-60 Hz. The device may comprise a cable and/or plug for connection to a mains supply.

Additionally or alternatively, the monitoring device may comprise one or more batteries (such as one or more rechargeable batteries) or a location for inserting one or more batteries in the device, such that the device may be powered by one or more batteries. The one or more batteries may provide power up to 2 W, for example.

The monitoring device may comprise an accessory which may be connected (e.g. via a wire or cable) to the rest of the monitoring device. The accessory may comprise of one or more optical sensors for measuring biochemical and/or molecular data (e.g. as described above), e.g. at a patient's hand (e.g. a hand not monitored, or on an arm not monitored, by the rest of the device).

The monitoring device may comprise one or more means for performing quality control. A means for performing quality control may be arranged to perform one or more quality control checks. A quality control check may comprise checking a measurement and/or signal output taken (measured) by one or more sensors of the device to ensure that the measurement and/or signal is adequate for analysis. Quality control is preferably performed prior to pre-processing (e.g. as described below). If a measurement and/or signal output from the one or more sensors is not found to be adequate for analysis (e.g. by the means for performing quality control), then that measurement and/or signal may be discarded, and, preferably, the user of the device may be guided to take another measurement (e.g. the device may indicate to a user that a/the measurement and/or signal output from the one or more sensors is not found to be adequate for analysis.

The monitoring device may comprise one or more sensors for measuring signal noise.

The monitoring device may comprise one or more processors arranged or adapted to perform pre-processing (e.g. one or more pre-processors). Pre-processing may comprise adjusting a measurement(s), e.g. to maximise or improve signal quality (preferably prior to analysing the data). This pre-processing or adjusting could comprise: filtering out unwanted signal(s), removing measurement (or signal) noise (e.g. as measured by one or more sensors for measuring signal noise), and/or adjusting for other (possibly interfering) effects such as skin properties (e.g. as measured by the device). This process could thus be based (at least partly) on signal noise measurements (e.g. as measured by the one or more sensors for measuring signal noise) and/or skin property data (e.g. as measured by the device).

The monitoring device may comprise (additionally or alternatively to the one or more processors arranged or adapted to perform pre-processing) one or more signal processors for performing signal processing. One or more signal processors may (also) perform quality control checks, for example. Signal processing may comprise taking a signal measured by one or more of the one or more sensors in the device and processing the signal to provide a (clinically useful) measurement (e.g. one which may be used for subsequent analysis and/or interpretation). In some embodiments, signal processing may comprise performing pre-processing as described above. In other embodiments, signal processing may not comprise performing pre-processing (e.g. this may be performed separately as described above).

The monitoring device may comprise a calibrator for performing calibration. The calibrator may be provided in the form of a component and/or tool (e.g. software installed on a component). The calibrator may be within or attached or connected to the monitoring device, or it may be a stand-alone component. Calibration (e.g. as performed by the calibrator), may comprise checking that a measurement to be taken by one or more sensors of the device can be acquired correctly and/or that it will represent the true output of that sensor (e.g. based on alignment with a known standard or source). Calibration (preferably or each of the one or more sensors in the monitoring device, or at least one or some of them) is preferably performed prior to a measurement being taken by the monitoring device.

The monitoring device preferably comprises means for analysing the patient data (e.g. analysis software provided on the device). The monitoring device preferably further comprises means for interpreting the patient data, or means for interpreting an analysis of the patient data.

The device preferably further comprises means for displaying a result of an analysis or interpretation of the patient data (e.g. as performed by the means for analysing or interpreting the patient data). The result could be or comprise an indication of the patient's health.

An initial assessment may be performed by the monitoring device at lower resolution (e.g. than a later assessment) and assist in the estimation of disease and detecting other abnormalities and/or complications. A later assessment may be performed at a higher resolution and may utilise a larger power supply and/or more advanced optical sensor(s), which may more accurately characterise and measure a wider range of conditions and assist in the identification of disease, detect other abnormalities and/or complications and/or patients with worsening symptoms.

According to a further aspect, there is provided a monitoring device for measuring patient (clinically useful) data, the monitoring device comprising one or more sensors for measuring electrical, mechanical, biochemical and molecular data. This device may have any of the features described above in relation to the first aspect.

According to a further aspect, there is provided a monitoring device for measuring patient (clinically useful) data, the monitoring device comprising: one or more sensors for measuring mechanical, electrical biochemical and/or molecular data; and one or more optical sensors for measuring biochemical and/or molecular data. This device may have any of the features described above in relation to the first aspect.

According to a further aspect, there is provided a method of measuring patient data from a patient, the method comprising applying a monitoring device according to any aspect described above (with any of its optional or preferred features) to the patient and using or controlling one or more of the one or more sensors to obtain patient data. The method preferably further comprises using the monitoring device to analyse the clinically useful data and, further preferably, to provide an analysis result.

The analysis result may provide an indication of a next step to be performed, such as:
- no further steps currently required
- further monitoring required, e.g. after a particular period of time, or
- (further) investigation or assessment from a health professional required (e.g. at a hospital or clinic).

The analysis result may, in some embodiments, be at least partially based on a location of the monitoring device.

The method preferably comprises the monitoring device providing an indication of the analysis result to a user, e.g. via a control panel or display.

The method may further comprise sending the clinically useful (patient) data from the monitoring device to a remote device, e.g. for storage or further analysis. This may only be possible when a suitable data connection is provided. The clinically useful (patient) data could be sent via a wired or wireless connection, for example.

The method may comprise receiving at the monitoring device data or software for use in a step of using the monitoring device to analyse the clinically useful (patient) data and provide an analysis result. As such, an analysis procedure performed at the device may be calibrated and/or updated based on data received or analysed from other devices or sources.

Preferred embodiments of the present invention may provide software which may enhance clinical workflows and/or escalate patient alerts, alongside the collection of clinically useful data which can support illness management, particularly where multiple infections, including opportunistic infections and neglected tropical diseases (NTDs), and non-infectious diseases or conditions coexist, but are not acted upon.

The applicant has developed a novel risk-category disease-specific technology strategy "track and trigger screening system" that can provide a comprehensive, non-invasive, intelligent medical diagnostic triage capability in a compact, portable, device, which may be linked, for example, to machine learning algorithms, allowing health systems to access certain features for individuals tested at facility-based or out-of-facility sites, or as part of healthcare worker-managed or client-managed groups.

Key features of preferred embodiments of the invention, offline and online, include equal and regular payment instalments and the ability to handle monitoring of clinically useful mechanical, electrical, biochemical and/or molecular data, as well as the ability to adjust for one or more skin properties, while synchronising measurements which could be expanded to other diseases, including as they emerge.

After a set of measurements has been taken (with at least some, if not all, of the various sensors in the device), algorithms (software) contained in one or more processors on the device may process the measured data to provide a health assessment, e.g. very rapidly. Once the device is connected via a data connection, local data from the device and/or software may be added to that already stored at a remote server, and latest algorithms/software may be downloaded from the remote server for (future) use at the device.

In addition to the facility-based or out-of-facility site applications, the present invention may have important spin-offs in drug development and in crisis and non-crisis public health situations.

According to embodiments of the present invention, a monitoring device may provide a portable, non-invasive, mechanical, electrical, and/or optical device, which is designed to be used primarily on the lower arm (below the elbow), hands and/or finger(s) of a patient and may include a small accessory for the (other) hand.

The device may be capable of working via battery (including rechargeable) or mains power.

All sensors may fit into and/or can be packed into a single, easy-to-clean housing to maximise infection control. The (single) housing is preferably designed to offer maximum protection to the inner components of the device whilst preferably accommodating any arm with an elbow-to-wrist length of 12 cm or longer.

The device may include an accessory in a separate housing for an optical light source and/or sensor that may be attached and/or connected to the main housing for additional functionality.

A screen and control panel may be contained within the housing. This may allow for an operator, e.g. with little or no medical experience and/or minimal training, to control the device.

The device preferably contains embedded operating software, transaction processing and/or on-device (analysis) software, which may enable the analysis of the clinically useful (patient) data measured by the device, e.g. thorough automated analysis protocols, to provide diagnostic and triage support.

The software may combine optics-based clinically useful measurements (e.g. skin property/properties, particularly skin phototype data) to allow for adjusting a measurement to account for different skin property/properties and/or different melanin (skin pigmentations) and/or collagen concentrations, and may be capable of safely communicating and sharing data with software located on a server.

Preferred embodiments of the invention will now be described by way of example only and with reference to the accompanying figures, in which:
Fig. 1 is a schematic diagram of a monitoring device; and
Fig. 2 is a schematic diagram of a software system that can be used in or in conjunction with the monitoring device.

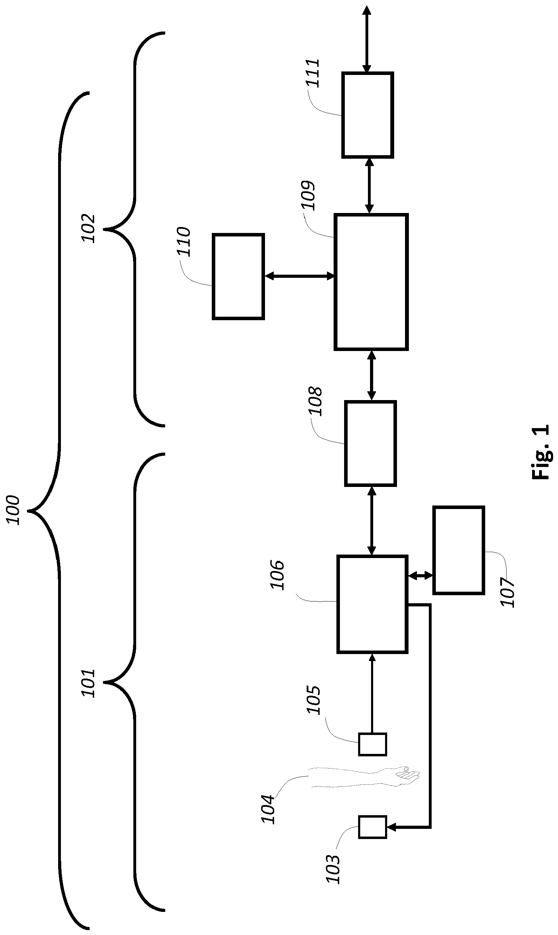

A monitoring device 100 according to one embodiment of the invention is illustrated in Fig. 1.

The monitoring device 100 is a portable, medical, mechanical, electrical and/or optical device. It is designed to be used primarily on a patient's lower arm (below the elbow), hands and/or finger(s).

In some embodiments, the monitoring device includes an accessory that can be attached, stand-alone and/or connected to the rest of the monitoring device 100 for additional functionality. This accessory is arranged and configured to make measurements on a patient's hand.

The monitoring device 100 can measure, monitor, detect and/or assess a number of different clinically useful data.

The monitoring device 100 comprises sensor(s) 101 and a main body 102.

The sensor(s) 101 are provided and arranged on the main body 102 in such a way as to accommodate and take measurements from a patient's lower arm (below the elbow), hands and/or finger(s). The main body 102 is arranged and configured to support and take measurements from a patient's lower arm (below the elbow), hands and/or finger(s).

The sensor(s) 101 may comprise one or more emitter(s) 103 which each act as a source of mechanical, electrical and/or optical detection transmitted to a measurement site 104 on a patient. The sensor(s) 101 may also comprise one or more detector(s) 105, which capture and measure mechanical, electrical, biochemical and/or molecular outputs from the measurement site 104.

Measurements made by the emitter(s) 103 and detector(s) 105 may be in combination (at the same time) or sequential, or a combination of both (e.g. some measurements may be made at the same time and some may be sequential).

The sensor(s) 101 of the monitoring device 100 also (each) include MCUs/processor(s) 106 and memory bank(s) 107. The MCUs/processor(s) 106 can control the emitter(s) 103 and/or detector(s) 105 and adapt (e.g. pre-process as described above) and process (e.g. as described above) the signal received so that it can be analysed by the monitoring device 100 and on-device software 202 (which is described below). As also shown in Fig. 1, memory bank(s) 107 are provided in the sensor(s) 101, which serve as a data storage location(s) for the MCUs/processor(s) 106, among other uses. The memory bank(s) 107 and MCUs/processor(s) 106 contained in the monitoring device 100 are for controlling the measurement/monitoring process, storing the measured data, and analysing clinically useful measurements taken by the monitoring device 100.

In some sensor(s) 101, the detector(s) 105 and MCUs/processor(s) 106 may collect and adapt (e.g. pre-process) skin property/properties signal(s) or data received by one or more of the detector(s) 105, such that it can be adjusted according to a range of tissue types (e.g. different skin pigmentations).

The monitoring device 100 comprises one or more outer casings which are designed to protect the sensor(s) 101 (each comprising emitter(s) 103, detector(s) 105, memory bank(s) 107, and micro-control unit(s) (MCUs)/processor(s) 106). The sensor components 103, 105, 107 and 106 are provided or stored inside the monitoring device 100.

The monitoring device 100 is sized and configured to accommodate a patient's arm with an elbow-to-wrist length of 12 cm or longer.

The monitoring device 100 also comprises in its main body 102 communication means (or interface 108 and/or 111) for communicating, e.g. wirelessly and/or via a wired connection, with on-device software and/or a server/software. Communicating with on-device software and/or a server/software may comprise sending and/or receiving data.

The main body 102 comprises a user interface 110 contained within the monitoring device 100. However, in alternative embodiments the monitoring device 100 can be provided without a user interface 110 and can provide an output to another display or system. The main body 102 can include various other components such as a microprocessor 109 or other components not illustrated in Fig. 1.

The user interface 110 allows an operator to control the monitoring device 100. In some embodiments, the user interface 110 comprises buttons, a touch screen display, a liquid crystal display (LCD), or the like for controlling the monitoring device 100. The user interface 110 may allow a status (e.g. battery level, monitoring status) of the monitoring device 100 and control options to be displayed to a user, e.g. for selection via the user interface 110 or to perform other operations (e.g. control data communications).

An interface 108 is provided between the sensor(s) 101 and the components of the main body 102, e.g. microprocessor 109. To communicate and/or share the mechanical, electrical, biochemical and/or molecular data between the sensor(s) 101 and the main body 102, the interface 108 can be a wired connection e.g. serial bus port and/or universal serial bus port (USB).

The user interface 110 communicates with the sensor(s) 101 via the microprocessor 109 and interface 108. The user interface 110 can also communicate with further, e.g. remote, devices via the microprocessor 109 and interface 111.

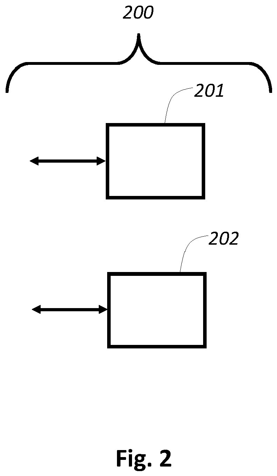

A software system 200 is provided for analysing data measured with the monitoring device 100. The software system 200 is illustrated in Fig. 2.

As illustrated schematically in Fig. 2, the software system 200 includes at least one server 201 with server software. The server 201 can store data (including data measured by the monitoring device 100), communicate with on-device software 202, generate and/or distribute software updates and automatic analysis protocols using, for example, medical-mathematical models, look-up tables, statistical learning models and/or neural network(s), monitor device performance data and/or generate outputs.

The software system 200 may also include on-device software 202. The on-device software 202 is provided on a monitoring device 100 (e.g. in the microprocessor 109) and may include software(s) to control the device 100, generate outputs, distribute the measurement and/or analysis of clinically useful data, and/or assess clinically useful data including through one or more automated analysis protocols. Communication to and from the on-device software 202 and/or between the various components of the system (e.g. server 201) may take place over a communications network such as the internet.

In some embodiments, the monitoring device 100 comprises one or more measurement site 104 light occlusion covers. Such covers can shade a measurement site 104 from ambient light. In use, a measurement site 104 may be located in a darkened or shaded region under this cover(s) so that measurements (e.g. optical measurements) requiring a low light-level environment can be taken.

The one or more sensor(s) 101 in the monitoring device 100 may comprise one or more mechanical, electrical and/or optical sensor(s) arranged to measure clinically useful data such as mechanical, electrical, biochemical and/or molecular data and/or skin property data.

The one or more optical sensors may comprise multi-wavelength optical sensor(s).

The optical sensor(s) provided in the monitoring device 4 each comprise an emitter(s) 103 and/or detector(s) 105 and are arranged to perform transmission, absorption and/or reflectance spectroscopy in the UV, visible, near-infrared, mid-infrared and far-infrared ranges, (i.e. wavelengths of 10 nm - 1 mm). Clinically useful data can be measured (or determined from measurements made) from the wavelength-dependent measurement of light. Clinically useful data which may be determined from such optical sensors may include oxygen saturation; haemoglobin (Hb) concentration; Hb derivatives (oxygenated Hb, deoxygenated Hb, carboxyhaemoglobin, methaemoglobin); haematocrit; blood clotting; blood velocity; blood viscosity; blood flow rate; relative blood flow; oxygen consumption rate; platelet adhesion and/or platelet rigidity; water and/or other molecules and when quantified and/or combined may be used to measure a range of conditions.

In some embodiments, the monitoring device comprises one or more thermometers. The one or more thermometers may be used to measure the temperature at one or more measurement sites 104. These measurements may be used to determine a temperature gradient, perfusion and/or circulation across a patent's lower arm (below the elbow), hands and/or finger(s). This is physiological data. The thermometer provided as one or more sensors may be a digital thermometer.

One or more optical sensor(s) may be arranged to measure (or measure data which can be used to determine) one or more of the following skin property data: skin phototype; collagen; melanin; and/or skin hydration. These are skin/tissue data.

The position of the emitter(s) 103 and/or detector(s) 105 is changeable so that the device can be adjusted to accommodate different lower arm (below the elbow), hands and/or finger(s) lengths and can allow some/all/any light-based measurements made by the monitoring device 100 to be calibrated according to one or more measured skin property/properties data.

Skin humidity may also be measured at the lower arm (below the elbow), hands and/or finger(s) with a moisture or humidity sensor provided in the monitoring device 100.

The position of the emitter(s) 103 and/or detector(s) 105 is changeable so that the device can be adjusted to accommodate different lower arm (below the elbow), hands and/or finger(s) lengths.

The monitoring device 100 may also comprise a cardioelectrical and/or cardiomechanical sensor(s) and body composition sensor(s).

One or more cardioelectrical and/or cardiomechanical sensor(s) and body composition sensor(s) can be provided in the small accessory for the hand described above.

The one or more cardioelectrical and/or cardiomechanical sensor(s) are arranged to measure the flow of electricity when cardiac cells produce a wave of depolarisation that can be sensed across the entire vascular tree. These sensor(s) are arranged to derive clinically useful data related to electrical impulses from the heart and may detect abnormalities and/or complications. The data measured by these sensor(s) is cardioelectrical and/or cardiomechanical data.

By including one or more sensor(s) for measuring cardioelectrical and/or cardiomechanical data in the monitoring device 100, along with sensors 101 for measuring other kinds of clinically useful data (e.g. as described above), cardioelectrical and/or cardiomechanical data may then be used in an analysis of and involving the other kinds of clinically useful data, allowing such an analysis to be improved compared, for example, with situations where cardioelectrical and/or cardiomechanical data are not available.

The one or more body composition sensor(s), which may be provided in the small accessory for the hand, comprise a source of alternating electrical current of very low intensity, which is arranged to be sent from metal electrodes through the patient's body so that the ability of biological tissues to impede the electrical current can be detected and measured. The body composition sensor(s) are arranged to measure lean mass and fluid volumes (e.g. total body water, intracellular fluid and/or extracellular fluid). The body composition sensor(s) may also be used to measure abnormal loss in lean body mass, the unbalanced shift in body fluids (e.g. severe dehydration and/or water retention), breathing and/or clinically useful data related to the timing and magnitude of the arterial wave.

This/these cardioelectrical and/or cardiomechanical sensor(s) and body composition sensor(s) can be provided in a region on the monitoring device 100 where, in use, a patient's measurement site 104 would be located, so that it/they can perform the above measurements on the patient's lower arm (below the elbow), hands and/or finger(s).

The monitoring device 100 is arranged to support and take measurements from a patient's lower arm (below the elbow), hands and/or finger(s).

The monitoring device 100 may comprise one or more thermometer(s), described above, to measure the patient's temperature at at least two different locations. The thermometer(s) may be digital thermometer(s).

The monitoring device 100 may further comprise one or more optical sensor(s), which is(are) arranged to measure a variety of biological molecules and/or disease-specific variations in molecular states on a patient's lower arm (below the elbow), hands and/or finger(s). Such optical sensor(s) can measure the unique spectral features representative of disease(s) in blood, interstitial fluid and/or tissue. Such measurements could be used to assess or detect life-threatening infections and/or cardiometabolic conditions. Such measurements may be or comprise biochemical and/or molecular data.

The position of the optical emitter(s) 103 and/or detector(s) 105 in the device 100 can be changeable so that the device can be adjusted to accommodate different lower arm (below the elbow), hands and/or finger(s) lengths.

One or more of the emitter(s) 103 and/or detector(s) 105 (or sensors 101) may be outside the device casing(s) and attached by wires and/or tubes at a fixed length and may be designed to be stored in the one or more device casing(s). Other sensor(s) 101 may be provided inside one or more device casings.

The monitoring device 100 may comprise cardioelectrical and/or cardiomechanical, body composition and/or mechanical sensor(s) and/or emitter(s) and detector(s) provided in the form of one or more moveable parts such that it/they may be moveable with respect to the rest of the monitoring device 100.

The one or more mechanical sensor(s) can be an electromechanical sensor based on a strain gauge load cell to obtain strength measurement(s) electronically. In particular, such a sensor can be used, for example, to measure continuous muscle function and maximum grip strength. These measurements may be used to derive a patient's muscle function and muscle strength data.

One or more mechanical sensor(s) may be provided in the device 100 for measuring clinically useful data related to the timing and magnitude of the arterial wave. Such mechanical sensor(s) may comprise a cuff in the form of an oscillometric and/or inflatable cuff. An inflation means (e.g. a pump and/or bulb) for the cuff may be provided to allow for manual inflation of the cuff. The cuff is arranged to make measurements of a patient's lower arm (below the elbow), hands and/or finger(s) lengths. In particular, the cuff is arranged to measure the travelling wave caused by cardiac contraction and reflection back from the periphery, which is influenced by the mechanical properties of the arteries, directly reflecting the status of the aorta, that may change early in the course of a disease. Such measurements may be vascular, cardiovascular, cardiac and/or aortic data.

The cuff and inflation means may be provided outside of the device casing in the form of a moveable part. They may be attached or otherwise connected to a patient appropriately and used for measuring, detecting and/or assessing clinically useful measurements.

An on/off function (e.g. switch) may be provided on the monitoring device 100 and an emergency off function (e.g. switch) may be provided. The emergency off function may allow for the monitoring device 100 to be turned off quickly in case of an emergency. The emergency off function is different from the on/off function as it acts as an emergency shutdown of all systems and power in/to the device. In some embodiments, the emergency off function may be incorporated into the on/off function, depending on the design of the user interface 110 and what it does. However, in other embodiments the on/off function and the emergency off function are provided as separate functions because, amongst other reasons, it can then be easier to notice the emergency off function if an emergency were to occur.

An interchangeable battery may be provided for powering the monitoring device 100. However, the device 100 can also (or alternatively) be powered by a mains electricity supply.

The monitoring device 100 comprises a number of memory banks 107 and MCUs/processors 106 for controlling the measurement, detection and/or assessment process, and storing measured data.

The monitoring device 100 comprises a number of different clinically useful sensor(s) 101 (e.g. as described above) which include mechanical, electrical and/or optical sensors.

The monitoring device 100 can connect via an interface 111 to on-device software 202. The on-device software 202 is software provided on the device 100 for analysing the clinically useful measurements taken by the various clinically useful sensors 101. The on-device software 202 can use automated analysis protocols derived from e.g. mathematical medical models, look-up tables, statistical learning models and/or neural network(s), to analyse the clinically useful data and provide diagnostic and triage support. The on-device software 202 is also capable of safely communicating with a server 201 (e.g. central server/software) when connected to an appropriate network.

In particular, the on-device software 202 can be arranged to use the clinically useful measurements to allow for adjustment according to, and the accommodation of, different skin property/properties (as measured by the device 100).

The technology provided on the monitoring device 100 features one or more sensor(s) 101 which contain emitter(s) 103 and/or detector(s) 105 for making mechanical, electrical and/or optical measurements of a patient. The sensor(s) may include a cuff, particularly an oscillometry and/or inflatable cuff, one or more optical, preferably multi-wavelength, sensors, such as but not limited to a UV, visible, near-infrared, mid-infrared and/or far-infrared transmission, vibrational, absorption and/or reflectance spectroscopy sensors, a white (LED) optical sensor, and/or a green (LED) optical sensor, such as a PPG sensor, on or more cardioelectrical and/or cardiomechanical monitor(s) such as a 1-lead, 6-lead or 12-lead ECG monitor, one or more preferably digital temperature sensors (or thermometers), a humidity sensor, a body composition sensor (or a pair of such sensors), and/or a preferably digital mechanical sensor for measuring muscle function.

MCUs/processor(s) 106 in each of the sensor(s) 101 are provided and are arranged to control the emitter(s) 103 and/or detector(s) 105, and to pre-process, process and/or analyse the signal received.

A further microprocessor 109 is provided in the main body 102 as an overall processor, e.g. for processing the measurements/results from the one or more MCUs/processors 106 provided for the one or more emitter(s) 103 and/or detector(s) 105. The MCUs/processors 106 and the microprocessor 109 are connected by the interface 108.

This multiple MCU and multiple memory bank architecture (e.g. at least one MCU 106 and memory bank 107 for each sensor 101, and also the microprocessor 109 in the main body 102) can help to mitigate against complete system loss. Data can be stored using the memory bank(s) 107, which allows for separate and distinct memory banks. Data from each emitter(s) 103 and/or detector(s) 105 can be stored in its respective memory bank 107 and backed-up to the other memory banks 107 (memory banks of other sensor(s) 101). Such a system can thereby provide a redundant array of independent memory banks 107 with enhanced protection against data loss if one of the memory banks 107 fails.

Communication with (to and from) the monitoring device 100 and between the sensor(s) 101 and the main body 102 occurs via one or more interfaces 108, 111 and can be wireless and/or wired.

A user interface 110 can be used for displaying outputs and overall monitoring device 100 control. In addition, there are wired capabilities for data transfer e.g. serial bus port and universal serial bus (USB).

The monitoring device 100 is designed with a focus on the user experience.

The monitoring device 100 is battery operated (although may alternatively be powered by a mains supply) and equipped with a user interface 110 that guides an operator to control the device.

The MCUs/processor(s) 106 may be of an ultra-low power variety with sleep mode(s) when not in use to help extend battery life between charging cycles.

Instead of a system where a device must always be connected online, offline measurement detection and assessment capabilities can also be provided. This can be particularly important in low- or middle-income countries, low resource settings, or places where trained operators are not available, where this device may be of particular use. Online, wireless or wired options may provide more accessible characterisation and measurement of a wide range of conditions, for example where a health system's use may assist in the identification of disease, detect other abnormalities and/or complications and/or patients with worsening conditions, including in remote locations.

The sensors 101 and other parts of the monitoring device 100 may be easily and quickly cleaned, e.g. with a sanitising wipe, ready for use on a further patient.

The monitoring device 100 can generate multiple clinically useful measurements and provide an output in real-time, i.e. directly after a set of measurements has been made on a patient. Using medical mathematical models, look-up tables, statistical learning models and/or neural network(s) to extract clinically useful data characteristics can allow the device 100 itself to provide an analysis result without the need for human analysis or interpretation of the measured data. Knowing when to trigger an escalation of cases, including referral to more intensive screening or to a higher level of onward care, can be service-specific and local. In addition, the software 202 is tailored to identify additional clinically useful measurements without the need for hardware modification.

The monitoring device 100 and software system 200 have various potential applications including:
- field-testing and clinical diagnostics;
- health care industry;
- remote/harsh environments;
- biopharmaceutical industries;
- screening and surveillance;
- military and disaster response;
- humanitarian and conflict situations;
- animal health;
- space missions.

All sensor(s) 101 on the monitoring device 100 are positioned and have dimensions designed to account for the maximum anthropometric range in hand and arm sizes. The sensors 101 can be positioned in the ideal (or close to ideal) anatomical locations on a patient's lower arm (below the elbow), hands and/or finger(s).

A smaller or simpler version of the monitoring device 100 may also or alternatively be provided. Such a device could comprise a different combination of sensor(s) 101 so would be lighter and simpler because of this. However, this device does comprise all of the sensor(s) 101 described above with respect to the monitoring device 100.

Dimensions of the device 100 may vary based on technological and usability requirements.

The casings of the monitoring device 100 can be made of plastic.

The monitoring device 100 has been designed to maximise safety and usability for both the user (operator) and the patient, therefore mitigating as much risk as possible, through the following features:
- Mechanical breakage
   ∘ The casing(s) are designed to be simple.
   ∘ In one embodiment, additional casings may be included to provide: space for desired components, noise reductions or limits and/or heat reductions or limits.
- Optical radiation
   ∘ A pressure sensor for the arm may be provided to block out the optical radiation if the sensor window is exposed.
   ∘ Sensor calibration prior to measurement acquisition (e.g. as performed by a calibrator) can provide the opportunity to derive a more accurate measurement(s).
- Electrical failure
   ∘ No mechanical openings to the casing(s), both during use and when not in use, to prevent ingress of dirt and water. All wires, cables and other connectors can be at a fixed length on the outside.
- Injury to user or patient
   ∘ The monitoring device 100 can be tested to IEC 60601 standards.
- Infection control
   ∘ Outer casing(s) can be designed to allow for easy cleaning.
   ∘ Outer casing(s) could allow for easy identification of any dirt (e.g. the casing(s) may be white or light-coloured).
- Stability
   ∘ Rubber feet provided on a bottom plate of the device may provide stability during use.
- General safety
   ∘ Easily identifiable and accessible emergency off function.
- Interface
   ∘ Sensors arranged to support and take measurements from a patient's lower arm (below the elbow), hands and/or finger(s).
- Usability
   ∘ Interchangeable battery may allow for longer lasting use.
   ∘ Possibility to be powered by mains allowing for longer lasting use.
   ∘ Easy set up and use, requiring minimal training.
   ∘ Anthropometric considerations incorporated into the design.

## Claims

1. A monitoring device for measuring patient data, the monitoring device comprising:
a. one or more sensors for measuring mechanical, electrical, biochemical and/or molecular data; and
b. one or more sensors for measuring skin property data.

2. A monitoring device as claimed in claim 1, wherein:
one or more of the one or more sensors for measuring mechanical data is/are arranged to measure haemodynamic data such as blood pressure, blood velocity, blood flow rate and/or relative blood flow; and/or
one or more of the one or more sensors for measuring mechanical, electrical and/or molecular data is/are arranged to measure cardiovascular data such as blood pressure, oxygen saturation, haemoglobin concentration, haemoglobin derivative(s), haematocrit, perfusion and/or circulation; and/or
one or more of the one or more sensors for measuring mechanical and/or molecular data is/are arranged to measure haemorheological data such as blood clotting, platelet adhesion, platelet rigidity, and/or blood viscosity.

3. A monitoring device as claimed in claim 1 or 2, wherein one or more of the one or more sensors for measuring mechanical, electrical, biochemical and/or molecular data, and/or one or more of the one or more sensors for measuring skin property data is/are arranged to measure patient data at a patient's lower arm, hand(s) and/or finger(s).

4. A monitoring device as claimed in claim 1, 2 or 3, wherein:
one or more of the one or more sensors for measuring mechanical, electrical, biochemical and/or molecular data comprise(s) a blood pressure monitor, one or more optical sensors and/or one or more body composition sensors; and/or
one or more of the one or more sensors for measuring skin property data comprise(s) one or more optical sensors.

5. A monitoring device as claimed in claim 4, wherein one or more of the one or more optical sensors is/are a spectrometer(s), optionally a vibrational spectrometer(s), and/or is/are arranged to operate at one or more wavelengths.

6. A monitoring device as claimed in claim 4 or 5, wherein:
one or more of the one or more optical sensors is/are arranged to measure mechanical and/or molecular data such as oxygen saturation, haemoglobin concentration, haemoglobin derivatives, haematocrit, platelet adhesion, platelet rigidity, blood clotting, and/or blood viscosity; and/or
one or more of the one or more optical sensors is/are arranged to measure biochemical data such as bilirubin level.

7. A monitoring device as claimed in claim 4, 5 or 6, wherein one or more of the one or more body composition sensors is/are arranged to measure fat mass, lean mass, total body water, intracellular fluid, extracellular fluid, phase angle, breathing and/or data related to timing and magnitude of a pulse wave.

8. A monitoring device as claimed in any preceding claim, wherein one or more of the one or more sensors for measuring skin property data is/are arranged to measure skin phototype data, collagen, melanin and/or skin hydration.

9. A monitoring device as claimed in any preceding claim, further comprising one or more sensors for measuring cardioelectrical and/or cardiomechanical data, the one or more sensors for measuring cardioelectrical and/or cardiomechanical data preferably comprising one or more electrical sensors.

10. A monitoring device as claimed in claim 9, wherein the one or more sensors for measuring cardioelectrical and/or cardiomechanical data is/are arranged to measure cardiac electrophysiology, cardiac and/or electrophysiology data at one or more locations on a patient's lower arm, hand(s) and/or finger(s).

11. A monitoring device as claimed in any preceding claim, further comprising a sensor for measuring muscle function and/or muscle strength data.

12. A monitoring device as claimed in any preceding claim, further comprising a positioning system for determining a position of the monitoring device.

13. A monitoring device as claimed in any preceding claim, further comprising one or more sensors for measuring signal noise.

14. A monitoring device as claimed in any preceding claim, further comprising a calibrator for calibrating one or more sensors.

15. A monitoring device as claimed in any preceding claim, further comprising means for analysing the patient data.

16. A method of measuring patient data from a patient, the method comprising applying a monitoring device as claimed in any preceding claim to the patient and using or controlling one or more of the one or more sensors to obtain patient data.

17. A method as claimed in claim 16, further comprising filtering out unwanted signal(s), removing signal noise, and/or adjusting for interfering effects such as skin properties.

18. The method of claim 16 or 17, further comprising using the monitoring device to analyse the patient data and provide an analysis result.

19. The method of any of claims 16 to 18, further comprising sending the patient data from the monitoring device to a remote device.

20. The method of any of claims 16 to 19, further comprising receiving at the monitoring device data or software for use in a step of using the monitoring device to analyse the patient data and provide an analysis result.

21. A monitoring device for measuring patient data, the monitoring device comprising one or more sensors for measuring electrical, mechanical, biochemical and molecular data.

22. A monitoring device for measuring patient data, the monitoring device comprising:
a. one or more sensors for measuring mechanical, electrical, biochemical and/or molecular data; and
b. one or more optical sensors for measuring biochemical and/or molecular data.
